(19) Europäisches Patentamt
European Patent Office
Office européen des brevets

(11) Publication number : 0 467 851 A1

# (12) EUROPEAN PATENT APPLICATION

(21) Application number : 91810562.8

(22) Date of filing : 11.07.91

(51) Int. Cl.$^5$ : **C07C 69/54,** C07C 57/075, C07C 15/46, C07C 67/62, C07C 7/20

(30) Priority : 20.07.90 US 556067

(43) Date of publication of application : 22.01.92 Bulletin 92/04

(84) Designated Contracting States : BE DE FR GB IT NL

(71) Applicant : **CIBA-GEIGY AG**
**Klybeckstrasse 141**
**CH-4002 Basel (CH)**

(72) Inventor : **Gatechair, Leslie R.**
**Rural Route 2, Box 60**
**Katonah, NY 10536 (US)**
Inventor : **Galbo, James P.**
**28 White Farm Road**
**Wingdale, NY 12594 (US)**
Inventor : **Ravichandran, Ramanathan**
**7 Kensington Court**
**Nanuet, NY 10954 (US)**

## (54) Stabilized monomer compositions.

(57) A process for stabilizing an ethylenically unsaturated monomer or oligomer from premature polymerization is disclosed whereby a stabilizing amount of an N-hydrocarbyloxy substituted hindered amine is added to said polymerizable monomer or oligomer. The ethylenically unsaturated monomer or oligomer encompass vinyl monomers or oligomers bearing at least one polymerizable moiety. The N-hydrocarbyloxy substituted hindered amine inhibits premature polymerization in the liquid and/or vapor phase.

EP 0 467 851 A1

The instant invention pertains to stabilized monomer compositions, in particular to compositions containing ethylenically unsaturated monomers, stabilized against premature polymerization.

The ethylenically unsaturated compounds which can be polymerized by free radical initiation are commonly called monomers. They constitute a major class of industrial chemicals. Because of the presence of the polymerizable double bond, the widespread sources of initiating radicals from peroxides, light and/or thermal generation, such monomers are prone to undesirable and premature polymerizarion at various stages during their manufacture, purification, storage, shipping, blending and use. Protection of such monomers from such premature polymerization is needed up to the point where polymerization is actually desired. If premature polymerization does occur, the monomer may suffer contamination by polymer, troublesome increase in viscosity, gelation and/or loss of reactivity. Fouling of distillation equipment including heat exchanger surfaces, storage vessels, transfer lines, pumps, shipping containers and application equipment can occur with ensuing costs of cleaning, downtime, loss of material and unnecessary labor costs. A particularly difficult situation is the preparation of polyol acrylates from polyols and acrylic acid since prolonged heating periods are required to complete the esterification. Premature polymerization can also constitute a safety hazard since uncontrolled exothermic polymerization can cause ruptured vessels, atmospheric contamination, and in extreme cases, explosions and fires. Deterioration of monomers in shipping and storage may also make necessary the use of costly refrigerated shipping and storage facilities.

A further problem is that of undesired polymerization of adventitious monomers, that is, radically-polymerizable unsaturated monomers which occur in commercial products such as hydrocarbon fuels and refinery streams. In these cases, polymerization accompanied by the incorporation of oxygen moieties leads to gum and sludge deposits which can foul carburetors, engines, fuel tanks or fuel lines. In refineries, the adventitious monomers in hydrocarbon streams such as cracking products can foul pipelines, valves, pumps, heat exchangers, stills and storage vessels.

Another problem in regard to undesired polymerizarion of free radical polymerizable monomers is the case of polymerizations which are intentional, but which must be prevented from going too far. For example, the quality of poly(vinyl chloride) suspension polymer and of synthetic rubber made from olefins and dienes is superior (i.e. better molecular weight distribution, stability, and processing properties) if the polymerization is stopped short of complete consumption of the monomers. It is also desirable to have available in a plant conducting vinyl polymerization reactions some rapid and efficient means for stopping a runaway polymerization if other means such as cooling should fail.

It is known that the addition of certain compounds to monomers can retard or even prevent their undesired polymerization, and that when polymerization of the monomer is desired, the inhibitor can be removed or overridden by a deliberately-added polymerization initiator. Various aromatic compounds have been used as such inhibitors in the prior art. Typical ones are hydroquinone, monomethyl ether of hydroquinone (MEHQ), tert-butylphenols, phenothiazine, phenylenediamines and benzoquinones. These are usually used at a level of 50 to 1000 ppm. These inhibitors are not totally effective and even with such inhibitors present, it is often advisable to store such inhibited monomers in a cool place and for limited periods of time. Moreover, these aromatic inhibitors are a cause of serious discoloration problems in the monomers and in polymers deliberately prepared from such monomers. Typically these aromatic inhibitors produce quinoidal chromophoric groups with very high visible light absorbance. The use of stable nitroxyl radicals as inhibitors also leads to discoloration since such compounds are themselves highly colored, usually bright red.

In order to overcome these color problems, a diligent search was made to find alternative inhibitors which are both effective and not discoloring. This search led to the N,N-dialkylhydroxylamines and the N,N-diaralkylhydroxylamines. Some typical references are cited infra.

US-A-3,222,334 and US-A-3,878,181 disclose the use of N,N-dialkylhydroxylamines such as N,N-diethylhydroxylamine as short-stopping agents for emulsion polymerizations of butadiene/styrene rubber and chloroprene.

US-A-3,148,225 and US-A-3,697,470 disclose the use of N,N-dialkylhydroxylamines such as N,N-diethylhydroxylamine and N-alkyl-N-arylhydroxylamine such as N-ethyl-N-phenylhydroxylamine respectively as short-stopping agents and popcorn polymer inhibitors in processes for preparing synthetic rubber. The popcorn polymer formation is a serious problem encountered in recovering of monomers from such synthetic rubber operations.

US-A-4,782,105 teaches the use of long chain N,N-dialkylhydroxylamines as stabilizers to prevent the premature gelation of unsaturated elastomer compositions such as styrene/butadiene copolymers or polybutadiene.

US-A-3,408,422 describes the use of N,N-dialkylhydroxylamines such as N,N-diethylhydroxylamine and N,N-diaralkylhydroxylamines such as N,N-dibenzylhydroxylamine as stabilizers for preventing the premature gelation of unsaturated polyesters.

US-A-4,798,889 teaches the use of N,N-dialkylhydroxylamines such as N,N-diethylhydroxylamine or N,N-dibenzylhydroxylamine as stabilizers to reduce the thermal polymerization of organosiloxanes substituted by ethylenically unsaturated moieties.

US-A-4,409,408 and US-A-4,434,307 disclose the use of N,N-dibenzylhydroxylamine in combination with an alkylated diphenol (catechol or hydroquinone) as inhibitors to prevent the polymerizarion of styrene.

The use of stable nitroxyl radicals including those derived from hindered amine moieties has also been disclosed. Typical references are cited below.

SU-A-1,139,722 describes the inhibition of styrene and comonomers such as butadiene using 1-oxyl derivatives of hindered amine compounds such as N,N′-bis-(1-oxyl-2,2,6,6-tetramethylpiperidin-4-yl)adipamide. The elimination of popcorn polymer and of the clogging of equipment is touted as the result of using such 1-oxyl compounds.

JP-A-60-36501 describes the use of hindered amines and their 1-oxyl and 1-alkyl derivatives as vinyl polymerization inhibitors to improve storage stability of monomers such as acrylate and methacrylate esters.

EP-A-178,168 and Brirish Patent No. 1,127,127 describe the use of 1-oxyl substituted hindered amine compounds as stabilizers for inhibiting the polymerization of $\alpha,\beta$-ethylenically unsaturated monocarboxylic acids, such as acrylic acid, during its recovery by distillation.

US-A-4,670,131 teaches the use of 1-oxyl substituted hindered amine compounds as stabilizers for preventing the fouling of equipment for processing organic feed streams containing olefins by inhibiting the polymerization of said olefins.

In a theoretical study of the inhibiting effects of selected hindered amine compounds, Y. Miura et al., Makromol. Chem. 160, 243 (1972) disclose that 1-oxyl-2,2,6,6-tetramethylpiperidin-4-one is highly effective in retarding the onset of the polymerization of styrene and methyl methacrylate. By contrast, the corresponding 1-benzyloxy-2,2,6,6-tetramethylpiperidin-4-one is stated to have no effect in delaying the polymerization of styrene and no retarding effect on said polymerization once begun.

US-A-4,668,721 and US-A-4,691,015 disclose the use of 1-hydroxy substituted hindered amine compounds as stabilizers for polyolefin compositions in combination with one or more other stabilizers such as phenolic antioxidants, ultraviolet light absorbers and the like.

None of these references describes or suggests that N-hydrocarbyloxy substituted hindered amine compounds are or could possibly be effective inhibitors to prevent the premature polymerization of monomers in either the liquid or vapor phase.

It is the broad object of the invention to provide monomer compositions inhibited against undesired and premature polymerization by means of small, but effective amounts of selected additives which do not impart undesired color to the monomer compositions.

It is a further object of the invention to provide inhibited monomer compositions which have substantially improved stability relative to compositions inhibited by methods known in the prior art.

It is a further object of the invention to provide a means for short-stopping or retarding polymerization of monomers once polymerization is started.

It is a further object of the invention to provide effective inhibitors for monomers known to be difficult to inhibit such as acrylic acid.

It is still a further object of the invention to provide highly effective combinations of inhibitors for said monomers.

The invention pertains to a monomer composition, stabilized against premature polymerization, which comprises

(a) an ethylenically unsaturated monomer or mixture of monomers, polymerizable by free radical initiation, and

(b) an effective amount, sufficient to inhibit the premature polymerization of component (a), of a compound or mixture of compounds of any of formulae I to XVII

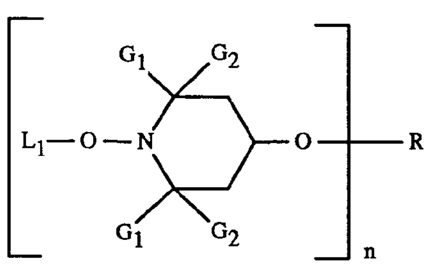

(I)

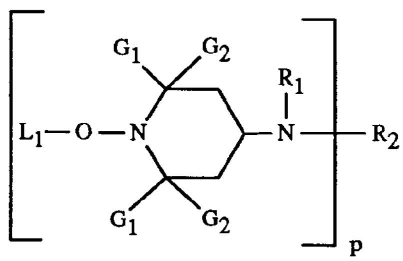

(II)

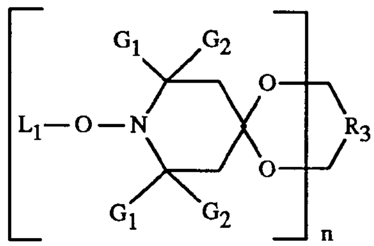

(III)

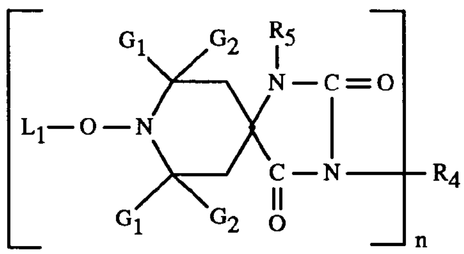

(IV)

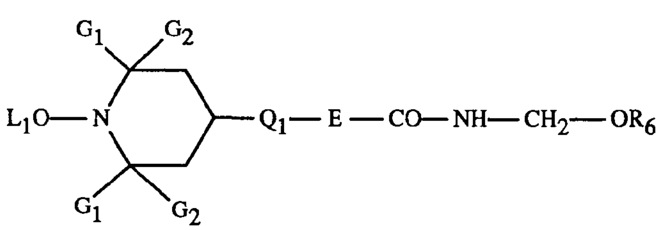

(V)

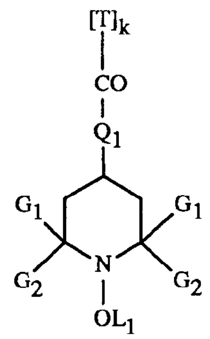

(VI)

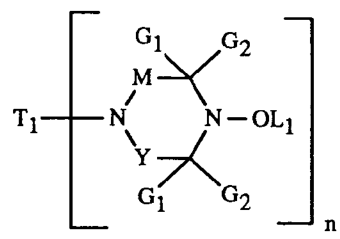

(VII)

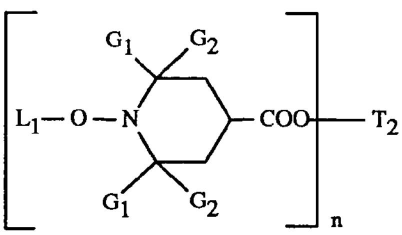

(VIII)

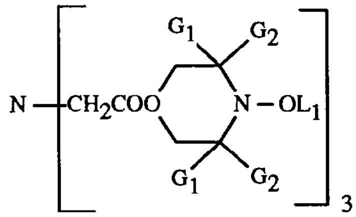

(IX)

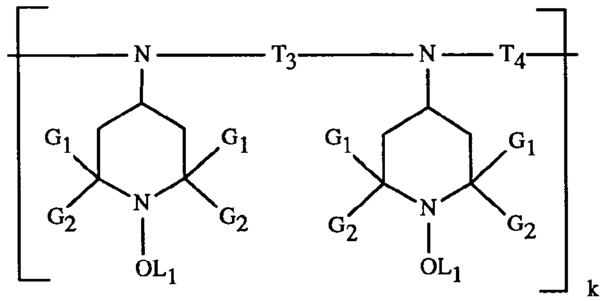

(X)

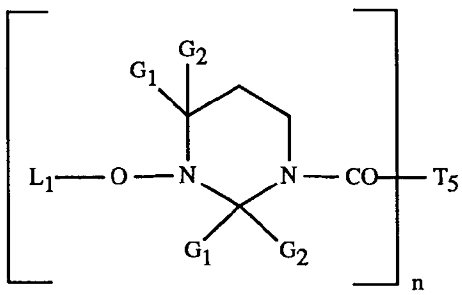

(XI)

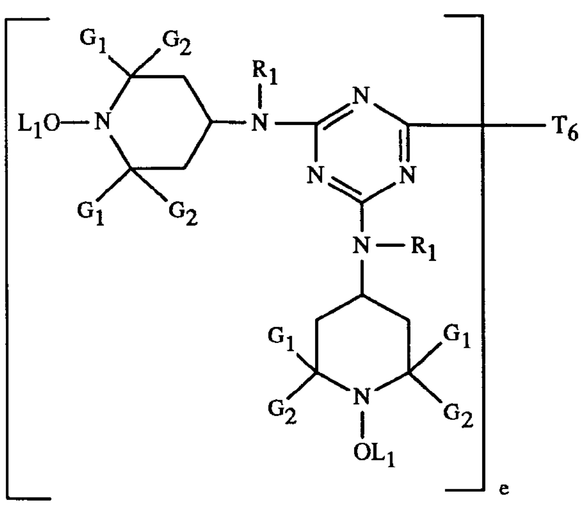

(XII)

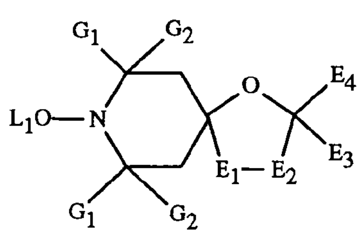

(XIII)

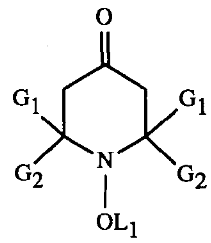

(XIV)

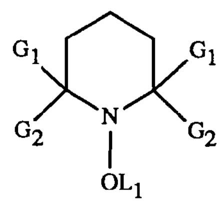

(XV)

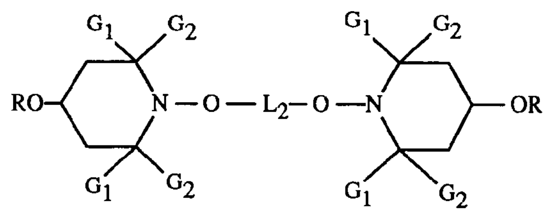

XVI

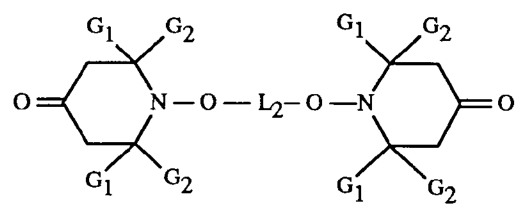

XVII

wherein

$G_1$ and $G_2$ are independently alkyl of 1 to 4 carbon atoms, preferably methyl, or $G_1$ and $G_2$ together are pentamethylene;

$L_1$ is alkyl of 1 to 18 carbon atoms, cycloalkyl of 5 to 12 carbon atoms, alkenyl of 2 to 18 carbon atoms, cycloalkenyl of 5 to 12 carbon atoms, aralkyl of 7 to 15 carbon atoms, a radical of a saturated or unsaturated bicyclic or tricyclic hydrocarbon of 7 to 12 carbon atoms or aryl of 6 to 10 carbon atoms or said aryl substituted by alkyl; or $L_1$ is $-CH_2CH_2COOL_3$ where $L_3$ is alkyl of 1 to 18 carbon atoms;

n is 1 or 2,

when n is 1,

R is hydrogen, $C_1$-$C_{18}$-alkyl optionally interrupted by one or more oxygen atoms, cyanoethyl, benzyl, glycidyl, a monovalent acyl radical of an aliphatic, cycloaliphatic, araliphatic or aromatic acid, or of carbamic acid or of a phosphorus-containing acid, or a monovalent silyl radical, preferably an acyl radical of an alipharic carboxylic acid having 2 to 18 carbon atoms, of a cycloaliphatic acid having 5 to 12 carbon atoms or of an aromatic carboxylic acid having 7 to 15 carbon atoms, or of carbamic acid; or

when n is 2,

R is $C_1$-$C_{12}$-alkylene, xylylene, a divalent acyl radical of an radical of an aliphatic, cycloaliphatic, araliphatic or aromatic dicarboxylic acid, or of a dicarbamic acid or of a phosphorus-containing acid, or a bivalent silyl radical, preferably an acyl radical of an aliphatic dicarboxylic acid having 2 to 36 carbon atoms, of a cycloaliphatic or aromatic dicarboxylic acid having 8 to 14 carbon atoms, or of a aromatic dicarbamic acid having 8 to 14 carbon atoms;

p is 1, 2 or 3,

$R_1$ is $C_1$-$C_{12}$-alkyl, $C_5$-$C_7$-cycloalkyl, $C_7$-$C_8$-aralkyl, $C_2$-$C_{18}$-alkanoyl, $C_3$-$C_5$-alkenoyl or benzoyl;

when p is 1,

$R_2$ is $C_1$-$C_{18}$-alkyl, $C_5$-$C_7$-cycloalkyl, $C_2$-$C_8$-alkenyl unsubstituted or substituted by a cyano, carbonyl or carbamide group, or is glycidyl, a group of the formula $-CH_2CH(OH)$-Z or of the formula -CONH-Z wherein Z is hydrogen, methyl or phenyl; or

when p is 2,

$R_2$ is $C_2$-$C_{12}$-alkylene, $C_6$-$C_{12}$-arylene, xylylene, a $-CH_2CH(OH)CH_2$-O-X-O-$CH_2CH(OH)CH_2$- wherein X is $C_2$-$C_{10}$-alkylene, $C_6$-$C_{15}$-arylene or $C_6$-$C_{12}$-cycloalkylene; or, provided that $R_1$ is not alkanoyl, alkenoyl or benzoyl, $R_2$ can also be a divalent acyl radical of an aliphatic, cycloaliphatic or aromatic dicarboxylic acid or dicarbamic acid, or can be the group -CO-; or $R_1$ and $R_2$ together when p is 1 can be the cyclic acyl radical of an aliphatic or aromatic 1,2- or 1,3-dicarboxylic acid; or

$R_2$ is

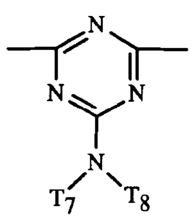

where $T_7$ and $T_8$ are independently hydrogen, alkyl of 1 to 18 carbon atoms, or $T_7$ and $T_8$ together are alkylene of 4 to 6 carbon atoms or 3-oxapentamethylene, preferably $T_7$ and $T_8$ are 3-oxapentamethylene;

when p is 3,

$R_2$ is 2,4,6-triazinyl;

when n is 1,

$R_3$ is $C_2$-$C_8$-alkylene or hydroxyalkylene or $C_4$-$C_{22}$-acyloxyalkylene; or

when n is 2,

$R_3$ is $(-CH_2)_2C(CH_2-)_2$;

when n is 1,

$R_4$ is hydrogen, $C_1$-$C_{12}$-alkyl, $C_3$-$C_5$-alkenyl, $C_7$-$C_9$-aralkyl, $C_5$-$C_7$-cycloalkyl, $C_2$-$C_4$-hydroxyalkyl, $C_2$-$C_6$-alkoxyalkyl, $C_6$-$C_{10}$-aryl, glycidyl, a group of formula $-(CH_2)_m$-COO-Q or of the formula $-(CH_2)_m$-O-CO-Q wherein m is 1 or 2 and Q is $C_1$-$C_4$-alkyl or phenyl; or

when n is 2,

$R_4$ is $C_2$-$C_{12}$-alkylene, $C_6$-$C_{12}$-arylene, a group $-CH_2CH(OH)CH_2$-O-X-O-$CH_2CH(OH)CH_2$- wherein X is $C_2$-$C_{10}$-alkylene, $C_6$-$C_{15}$-arylene or $C_6$-$C_{12}$-cycloalkylene, or a group $-CH_2CH(OZ_1)CH_2-(OCH_2CH(OZ_1)CH_2)_2$- wherein $Z_1$ is hydrogen, $C_1$-$C_{18}$-alkyl, allyl, benzyl, $C_2$-$C_{12}$-alkanoyl or benzoyl;

$R_5$ is hydrogen, $C_1$-$C_{12}$-alkyl, allyl, benzyl, glycidyl or $C_2$-$C_6$-alkoxyalkyl;

$Q_1$ is $-N(R_7)$- or -O-;

E is $C_1$-$C_3$-alkylene, the group $-CH_2CH(R_8$-O- wherein $R_8$ is hydrogen, methyl or phenyl, the group $-(CH_2)_3$-NH- or a direct bond;

$R_7$ is $C_1$-$C_{18}$-alkyl, $C_5$-$C_7$-cycloalkyl, $C_7$-$C_{12}$-aralkyl, cyanoethyl, $C_6$-$C_{10}$-aryl, the group $-CH_2CH(R_8)$-OH; or a group of the formula

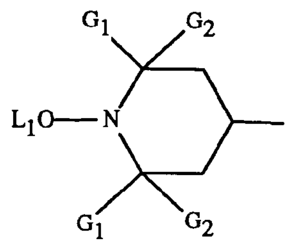

or a group of the formula

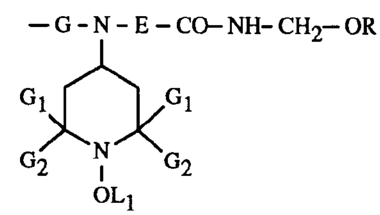

wherein G is $C_2$-$C_6$-alkylene or $C_6$-$C_{12}$-arylene; or

$R_7$ is a group -E-CO-NH-$CH_2$-$OR_6$;

$R_6$ is hydrogen or $C_1$-$C_{18}$-alkyl;

Formula VI denotes a recurring structural unit of a polymer where T is ethylene or 1,2-propylene, or is a repearing structural unit derived from an α-olefin copolymer with an alkyl acrylate or methacrylate, preferably a copolymer of ethylene and ethyl acrylate;

k is 2 to 100;

$T_1$ has the same meaning as $R_2$ when p is 1 or 2;

M and Y are independently methylene or carbonyl, preferably M is methylene and Y is carbonyl, and $T_1$ is ethylene when n is 2;

$T_2$ has the same meaning as $R_4$, and $T_2$ is preferably octamethylene when n is 2,

$T_3$ and $T_4$ are independently alkylene of 2 to 12 carbon atoms, of $T_4$ is

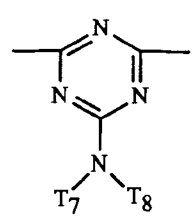

$T_6$ is

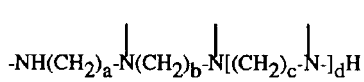

where a, b and c are independently 2 or 3, and d is 0 or 1, preferably a and c are each 3, b is 2 and d is 1;

e is 3 or 4, preferably 4;

$T_5$ is the same as R with the proviso that $T_5$ cannot be hydrogen when n is 1;

$E_1$ and $E_2$ being different, are each oxo or imino, preferably $E_1$ is oxo and $E_2$ is -N($E_5$)- where $E_5$ is $C_1$-$C_{12}$-alkyl or alkoxycarbonylalkyl of 4 to 22 carbon atoms;

$E_3$ is hydrogen, alkyl of 1 to 30 carbon atoms, phenyl, naphthyl, said phenyl or said naphthyl substituted by chlorine or by alkyl of 1 to 4 carbon atoms, or phenylalkyl of 7 to 12 carbon atoms, or said phenylalkyl substituted by alkyl of 1 to 4 carbon atoms;

$E_4$ is hydrogen, alkyl of 1 to 30 carbon atoms, phenyl, naphthyl or phenylalkyl of 7 to 12 carbon atoms; or $E_3$ and $E_4$ together are polymethylene of 4 to 17 carbon atoms, or said polymethylene substituted by up to four alkyl groups of 1 to 4 carbon atoms, preferably methyl; and

$L_2$ is an alkanediyl of 1 to 18 carbon atoms or cyclohexanediyl.

The monomers of component (a) of this invention are any having at least one carbon-carbon double bond capable of undergoing free radical induced polymerization. Such monomers are well known in commerce and comprise a wide variety of structural types. Typical examples of such monomers are the olefinic hydrocarbons such as styrene, α-methylstyrene and divinylbenzene; dienes such as butadiene and isoprene; halogenated monomers such as vinyl chloride, chloroprene, vinylidene chloride, vinylidene fluoride and vinyl fluoride; unsaturated acids such as acrylic acid, methacrylic acid and crotonic acid; unsaturated esters such as vinyl acetate, alkyl acrylates and alkyl methacrylates such as methyl methacrylate, ethyl acrylate, methyl acrylate, 2-hydroxyethyl acrylate and methacrylate, ethylene bismethacrylate, trimethylolpropane triacrylate, acrylated epoxy resin and polyethylene glycol diacrylate; unsaturated amides such as acrylamide, N,N-dimethylacrylamide, methylene-bisacrylamide and N-vinylpyrrolidone; unsaturated nitrile monomers such as acrylonitrile; and unsaturated ethers such as methyl vinyl ether; and miscellaneous monomers such as the vinyl pyridines, diethyl vinylphosphonate and sodium styrenesulfonate.

The instant invention also pertains to mixtures of said monomers and to resins such as acrylate-terminated polyurethanes and unsaturated polyesters. The common feature making all of these materials relevant to the present invention is the presence of a polymerizable double bond.

Also in the category of monomers are unsaturated oils such as drying oils like linseed oil, where polymerization also incorporates oxygen. There are also adventitious monomers formed in refining processes, for example polymerizable olefinic unsaturation in gasoline, jet fuel, solvents, crude oil and cracked hydrocarbon streams. The common feature of all of these substances is encompassed in the broad term "monomers" and all are contemplated to be within the scope of instant component (a). Polymerization of such materials is often accompanied by autooxidation.

The acrylates, particularly acrylic acid itself, are unusually difficult to inhibit because of their inherent high polymerizability. The instant compounds are shown to be particularly effective in inhibiting acrylic acid from premature polymerization.

Preferably component (a) is a monomer selected from the group consisting of the olefinic hydrocarbons, dienes, halogenated monomers, unsaturated acids, unsaturated esters, unsaturated amides, unsaturated nitriles, unsaturated ethers, acrylated urethanes and unsaturated polyesters and mixtures thereof.

Most preferably the monomer of component (a) is styrene, butadiene, vinyl chloride, acrylic acid, methacrylic acid, vinyl acetate, 2-hydroxyethyl aryclate, 2-hydroxyethyl methacrylate, trimethylolpropane triacrylate, polyethylene glycol diacrylate or methyl methacrylate.

Still more preferably the monomer is styrene, butadiene, acrylic acid or methacrylic acid.

The N-hydrocarbyloxy derivatives useful in the instant invention are denoted by the various structures of formulas I to XV. Most of these N-hydrocarbyloxy derivatives are known compounds. The instant N-hydrocarbyloxy derivatives can be easily prepared from the corresponding hindered amines which are known or which can be made by known procedures.

Particularly preferred compounds are those of formula I or XV, especially of formula I. Further very effective and preferred compounds are

1-α-methylbenzyloxy-2,2,6,6-tetramethylpiperidin-4-one;
1-α-methylbenzyloxy-2,2,6,6-tetramethylpiperidin-4-ol;
1-α-methylbenzyloxy-2,2,6,6-tetramethyl-4,4-ethylenedioxypiperidine;
1-tert-butoxy-2,2,6,6-tetramethylpiperidin-4-ol;
1-methylcyclohexyloxy-2,2,6,6-tetramethyl-4-benzoyloxypiperidine;
bis(1-octyloxy-2,2,6,6-tetramethylpiperidin-4-yl) sebacate;
1-[2-(methoxycarbonyl)ethoxy]-4-benzyloxy-2,2,6,6-tetramethylpiperidine; and
1-methoxy-4-benzoyloxy-2,2,6,6-tetramethylpiperidine.

The hydroxylamine derivative may generally be prepared by oxidizing a hindered amine with a peroxy compound such as hydrogen peroxide followed by reduction of the oxyl intermediate formed to the desired hydroxylamine derivative. Such a process is taught in US-A-4,665,185.

The instant N-hydrocarbyloxy derivatives are made by reacting a hydroxylamine with an alkyl halide or benzyl halide or reacting the hydroxylamine with an alkyl in presence of potassium butoxide.

Another method involves the preparation of the N-hydrocarbyloxy compounds directly from the hindered amine precursors using aqueous tert-butyl hydroperoxide, molybdenum trioxide in an appropriate hydrocarbon medium.

If any of the substituents are $C_1$-$C_{12}$-alkyl, they are for example methyl, ethyl, n-propyl, isopropyl, n-butyl,

sec-butyl, tert-butyl, n-amyl, tert-amyl, n-hexyl, n-octyl, 2-ethylhexyl, tert-octyl, n-nonyl, n-decyl, n-undecyl or n-dodecyl. As $C_1$-$C_{18}$-alkyl, R can be the aforementioned groups, and in addition for example n-tridecyl, n-tetradecyl, n-hexadecyl or n-octadecyl.

$L_1$ is preferably alkyl of 1 to 12 carbon atoms, cyclohexyl or alpha-methylbenzyl; most preferably methyl, heptyl, octyl, nonyl, cyclohexyl or alpha-methylbenzyl.

If R is a monovalent acyl radical of a carboxylic acid, it is for example an acyl radical of acetic acid, stearic acid, salicylic acid, methacrylic acid, acrylic acid, maleic acid, benzoic acid, 2-ethylhexanoic acid or 3,5-di-tert-butyl-4-hydroxyhydrocinnamic acid.

If R is a divalent acyl radical of a dicarboxylic acid, it is for example an acyl radical of adipic acid, succinic acid, suberic acid, sebacic acid, o-phthalic acid, butylmalonic acid, dibutylmalonic acid, dibenzylmalonic acid, 3,5-di-tert-butyl-4-hydroxybenzyl-butyl-malonic acid or bicycloheptene dicarboxylic acid.

If R is a divalent acyl radical of a dicarbamic acid, it is for example an acyl radical of hexamethylenedicarbamic acid or 2,4-toluylenedicarbamic acid.

R is also an acyl radical of a phosphorus-containing acid of the formula

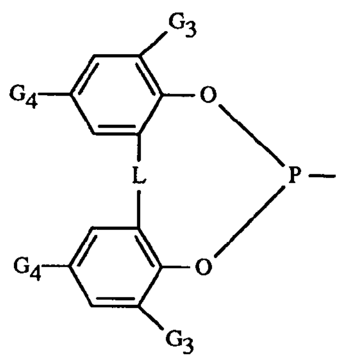

(D)

wherein L is a direct bond, methylene or alkylidene of 2 to 6 carbon atoms such as ethylidene, butylidene or amylidene. Preferably L is a direct bond, methylene or ethylidene.

$G_3$ and $G_4$ are independently alkyl of 1 to 4 carbon atoms, preferably methyl or tert-butyl. Most preferably $G_3$ and $G_4$ are each tert-butyl, or $G_3$ is tert-butyl and $G_4$ is methyl.

If any substituents are $C_5$-$C_7$-cycloalkyl, they are in particular cyclohexyl.

As $C_7$-$C_8$-aralkyl, $R_1$ is phenethyl and especially benzyl.

As $C_2$-$C_{18}$-alkanoyl, $R_1$ is for example propionyl, butyryl, octanoyl, lauroyl, hexadecanoyl, octadecanoyl, but especially acetyl; and as $C_3$-$C_5$-alkenoyl, $R_1$ is in particular acryloyl.

If $R_2$ is $C_2$-$C_8$-alkenyl unsubstituted or substituted by a cyano, carbonyl or carbamide group, it is for example 1-propenyl, allyl, methallyl, 2-butenyl, 2-pentenyl, 2-hexenyl, 2-octenyl, 2,2-dicyanovinyl, 1-methyl-2-cyano-2-methoxycarbonyl-vinyl or 2,2-diacetylaminovinyl.

When $R_1$ and $R_2$ are together a cyclic acyl radical, they are especially -CO-$(CH_2)_5$-.

If any substituents are $C_2$-$C_{12}$-alkylene, they are for example ethylene, propylene, 2,2,-dimethylpropylene, tetramethylene, hexamethylene, octamethylene, decamethylene or dodecamethylene.

If any substituents are $C_6$-$C_5$-alkylene, they are for example o-, m- or p-phenylene, 1,4-naphthylene or 4,4'-diphenylene.

As $C_6$-$C_{12}$-cycloalkylene, X is especially cyclohexylene.

If $R_3$ is $C_2$-$C_8$-alkylene or hydroxyalkylene, it is for example ethylene, 1-methyl-ethylene, propylene, 2-ethylpropylene or 2-ethyl-2-hydroxymethylpropylene.

As $C_4$-$C_{22}$acyloxyalkylene, $R_3$ is for example 2-ethyl-2-acetoxymethyl-propylene.

If any substituents are $C_2$-$C_6$-alkoxyalkyl, they are example methoxymethyl, ethoxymethyl, propoxymethyl, tert-butoxymethyl, ethoxyethyl, ethoxypropyl, n-butoxyethyl, tert-butoxyethyl, isopropoxyethyl or propoxypropyl.

If $R_4$ is $C_3$-$C_5$-alkenyl, it is for example 1-propenyl, allyl, methallyl, 2-butenyl or 2-pentenyl.

As $C_7$-$C_9$-aralkyl, $R_4$ is phenethyl or especially benzyl; and as $C_5$-$C_7$-cyclohexyl is especially cyclohexyl.

If $R_4$ is $C_2$-$C_4$-hydroxyalkyl, it is for example 2-hydroxyethyl, 2-hydroxypropyl, 3-hydroxypropyl, 2-hydroxybutyl or 4-hydroxybutyl.

As $C_6$-$C_{10}$-aryl, $R_4$ is in particular phenyl or $\alpha$- or $\beta$-naphthyl which is unsubstituted or substituted by halogen or $C_1$-$C_4$-alkyl.

If $R_4$ is $C_2$-$C_{12}$-alkylene, it is for example ethylene, propylene, 2,2-dimethylpropylene, tetramethylene,

hexamethylene, octamethylene, decamethylene or dodecamethylene.

If $R_4$ is $C_6$-$C_{12}$-arylene, it is for example o-, m- or p-phenylene, 1,4-naphthylene or 4,4′-diphenylene.

If $Z_1$ is $C_2$-$C_{12}$-alkanoyl, it is for example propionyl, butyryl, octanoyl, dodecanoyl or preferably acetyl.

As $C_5$-$C_7$-cycloalkyl, $R_7$ is particularly cyclohexyl.

As $C_6$-$C_{10}$-aryl, $R_7$ is particularly phenyl or α- or β-naphthyl which is unsubstituted or substituted with halogen or $C_1$ -$C_4$-alkyl.

As $C_1$-$C_3$-alkylene, E is for example methylene, ethylene or propylene.

As $C_2$-$C_6$-alkylene, G is for example ethylene, propylene, 2,2-dimethylpropylene, tetramethylene or hexamethylene; and as $C_6$-$C_{12}$-arylene, G is o-, m- or p-phenylene, 1,4-naphthylene or 4,4′-diphenylene.

Preferably $L_2$ is methylene, butanediyl, heptanediyl, octanediyl, nonanediyl, dodecanediyl or cyclohexanediyl; most preferably, octanediyl or cyclohexanediyl.

An effective inhibiting amount of an instant compound of this invention needed to retard or prevent premature free radical induced polymerization of a monomer or monomer mixture is preferably in the range of from 1 to 10000 ppm, based on the total monomer composition, with the more preferred range being 5 to 2000 ppm and the most preferred range being 50 to 1000 ppm. The lower amounts would be used where the degree of inhibition required is not great such as when the monomers are to be used promptly, or which will be stored refrigerated, or which are inherently less prone to polymerize readily such as monomers with internal double bonds. The higher amounts of inhibitor would be used where the monomer is to be stored for prolonged periods of time, especially under relatively warm conditions or where contamination is likely, or where exposure to photoinitiation is likely, or where the monomer is especially prone to rapid polymerization with little provocation such as with the acrylates and acrylic acid. Those skilled in the art of vinyl polymerization are well aware of the relative polymerizability of monomers and of their relative stabilities.

The stabilized compositions of this invention are distinguished by their lack of color.

The compositions of the instant invention may also contain additional inhibitors, such as hydroquinone, the monomethyl ether of hydroquinone, phenothiazine (these three often being required by monomer specifications) or catechol, tert-butylated hydroquinones or catechols, other alkylated phenols, nitrosophenols, nitrosophenylhydroxylamines, alkylated phenothiazines, sulfur and hindered cyclic amines or their corresponding oxyl derivatives.

The inhibited compositions may also contain metal deactivators and UV absorbers to improve light stability; or stabilizers such as amines to retard acid-catalyzed degradation; or thermal or photoinitiators; and other conventional additives.

When it is desired to subject the inhibited monomer to polymerization, the inhibitor can either be removed or overridden by sufficient polymerization initiator. Removal can be accomplished by distillation, absorption or washing with an acidic solution. It is possible to remove the instant 1-hydroxy derivatives while leaving the phenolic antioxidants in the monomer by use of strong acid ion exchange resins. The polymerization inhibiting action of the instant compounds can be overridden by use of sufficient free radical initiator, actinic light irradiation, electron beam exposure or other polymerization initiating means.

The instant invention also pertains to a process for preventing the premature polymerization of a monomer polymerizable by free radical initration which comprises adding to said monomer (a) an effective amount of any of the components (b) described above. The process of the instant invention involves simply dissolving an effective inhibiting amount of the inhibitor in the monomer prior to exposure of the latter to conditions where the premature, undesired free radical initiated polymerization might occur.

Preferably, this process comprises

adding 10 to 500 ppm of at least one compound of formula I to XVII to a continuous fluid feed stream to deactivate the autocatalytic polymerization, in any part of the continuous process equipment, such as reactor, reboiler, distillation column, etc., of any ethylenically unsaturated monomer present in the feed stream, and

further adding to said feed stream an additional 10 ppb to 500 ppm of at least one compound of formula I to XVII as a makeup additive to maintain the desired concentration of said compound in the fluid feed stream being processed.

Preferably, this process is also carried out to prevent the fouling of processing equipment including reactors, pipes, stills, distillation columns, cracking towers and heat transfer surfaces during the processing of a monomer polymerizable by free radical initration.

The following examples are presented for the purpose of illustration only and are not to be construed as limiting the instant invention in any manner whatsoever.

Example 1

Acrylic acid (105 g) to which is added 1000 ppm of the test additive is placed in a 100 ml flask equipped

with a condenser and nitrogen sparge tube and then heated in an oil bath at 150°C. The time in seconds from the onset of heating till polymer is observed to form on the side of the flask near the surface of the liquid or in the vapor phase on the sparge tube above the splash line of the liquid is recorded. These times are given in the table below.

| | Time in Seconds till Polymer is Seen | |
|---|---|---|
| Additive* (100 ppm) | On Flask Wall | On Sparge Tube |
| Compound A | 200 | 780 |
| Compound B | 200 | 530 |
| Compound C | 210 | 600 |
| Compound D | 290 | 700 |
| Compound E | 150 | 650 |
| Compound F | 210 | 600 |
| Compound G | 175 | 485 |

* Compound A is 1-α-methylbenzyloxy-2,2,6,6-tetramethylpiperidin-4-one.
Compound B is 1-α-methylbenzyloxy-2,2,6,6-tetramethylpiperidin-4-ol.
Compound C is 1-α-methylbenzyloxy-2,2,6,6-tetramethyl-4,4-ethylenedioxy-piperidine.
Compound D is 1-tert-butoxy-2,2,6,6-tetramethylpiperidin-4-ol.
Compound E is 1-cyclohex-2-enyloxy -2,2,6,6-tetramethylpiperidin-4-ol.
Compound F is 1-methylcyclohexyloxy-2,2,6,6-tetramethyl-4-benzoyloxy-piperidine.
Compound G is bis(1-octyloxy-2,2,6,6-tetramethylpiperidin-4-yl) sebacate.

When no inhibitor is present, the flask very rapidly fills with solid polymer.

Example 2

Liquid Phase Inhibition

To the monomer methyl methacrylate is added 100 ppm of the test additive 1-[2-(methoxycarbonyl)ethoxy]-4-benzyloxy-2,2,6,6-tetramethylpiperidine (Compound H). The stabilized monomer is then held at 80°C in a sealed bottle till polymerization of the monomer is observed visually. The time in hours till polymerization occurs is a measure of the effectiveness of the test additive as a monomer polymerization inhibitor. Polymerization occurs with unstabilized methyl methacrylate after 46 hours, but with the methyl methacrylate containing the test inhibitor no polymerization observed before 1425 hours.

Example 3

Vapor Phase Inhibition

Acrylic acid (105 g, 100 ml) is added to a resin kettle whose weight is known and which is fitted with a reflux condenser and nitrogen inlet tube. A stream of nitrogen at 250 ml/min is used to flush the kettle for 15 minutes. The kettle containing the acrylic acid is then immersed into a 6-liter oil bath such that the top of the acrylic acid solution is approximately 5 cm below the level of the oil surface. The kettle and its contents are heated at 150°C causing the acrylic acid to reflux. The kettle is cooled after only 20 minutes due to very rapid buildup of solid polymer in the lower region of the kettle. After washing with hexane, the kettle is found to contain 82.9 grams of solid polymer. No polymer is seen on the walls of the kettle in the vapor region presumably due to the rapid formation of polymer in the liquid phase area.

Using the procedure described above, 100 ppm by weight of two test polymerization inhibitors are individually added to acrylic acid and the above procedure repeated. The test solutions are refluxed for 100 minutes with the amount of polymer formed in the liquid and vapor phases being recorded after any residual monomer is removed by washing with hexane. These results are given in the table below.

| | Polymer Formed in Grams | |
|---|---|---|
| Additive* (100 ppm) | Liquid Phase | Vapor Phase |
| None | 82.9 | -- |
| Compound H | none | 52 |
| Compound I | none | 92 |

*Compound H is 1-[2-(methoxycarbonyl)ethoxy]-4-benzyloxy-2,2,6,6-tetramethylpiperidine; Compound I is 1-methoxy-4-benzoyloxy-2,2,6,6-tetramethylpiperidine.

Both instant compounds are very effective in preventing premature polymerization of acrylic acid in the liquid phase. Instant Compound H is particularly effective in preventing the vapor phase polymerization of acrylic acid.

## Claims

1. A monomer composition, stabilized against premature polymerization, which comprises
   (a) an ethylenically unsaturated monomer or mixture of monomers, polymerizable by free radical initiation, and
   (b) an effective amount, sufficient to inhibit the premature polymerization of component (a), of a compound or mixture of compounds of any of formulae I to XVII

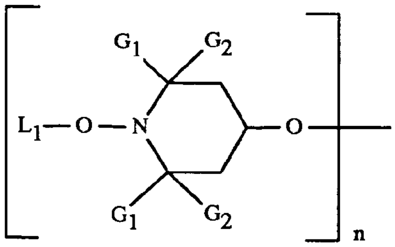

(I)

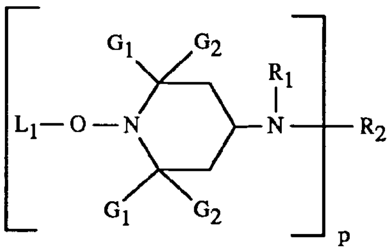

(II)

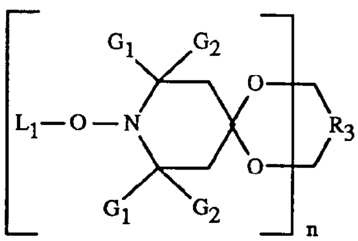

(III)

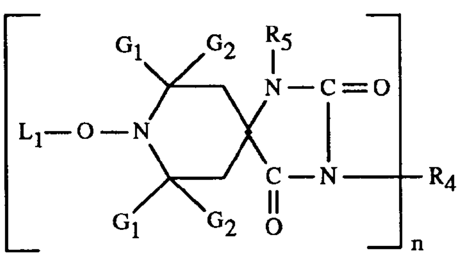

(IV)

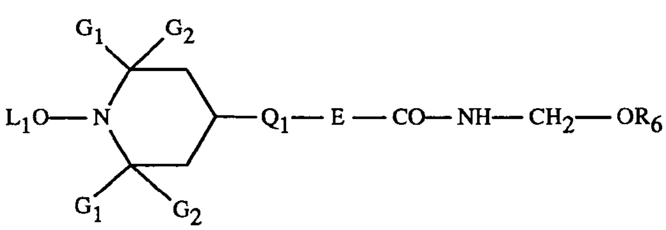

(V)

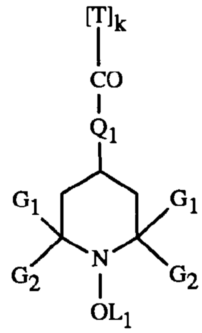

(VI)

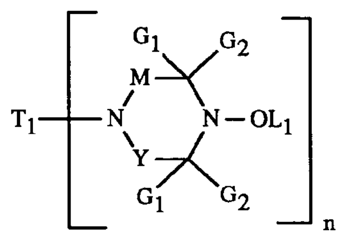

(VII)

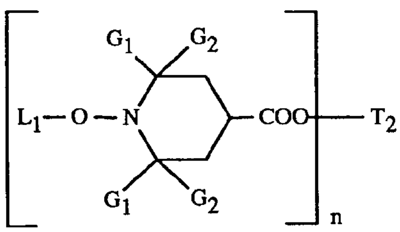

(VIII)

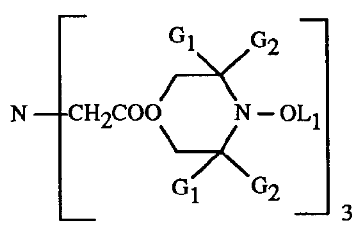

(IX)

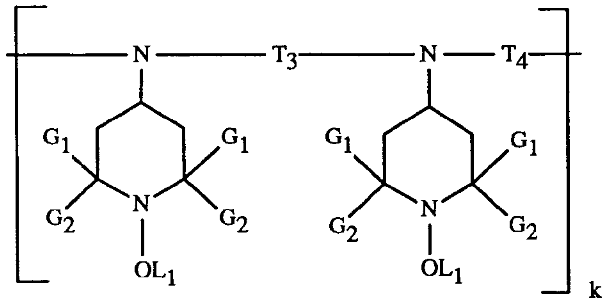

(X)

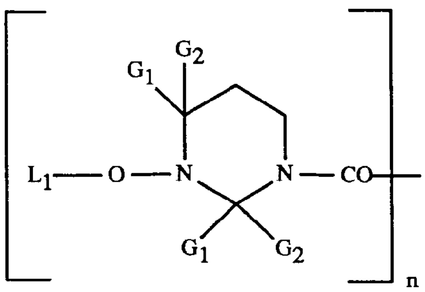

(XI)

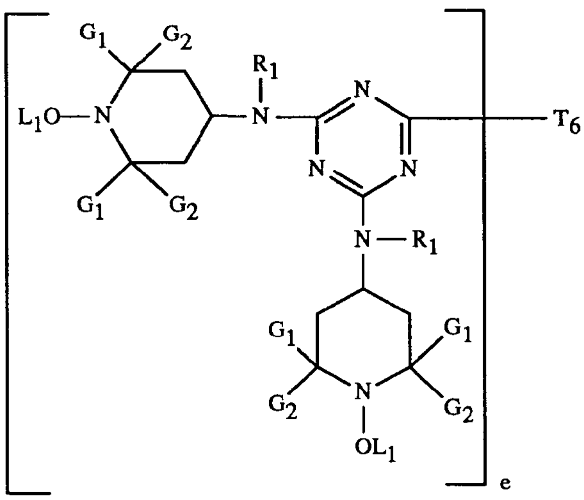

(XII)

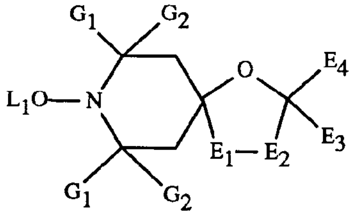

(XIII)

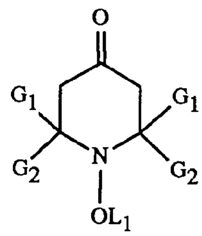

(XIV)

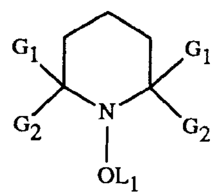

(XV)

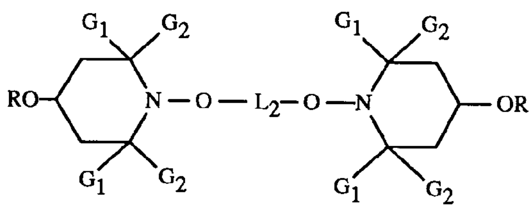

XVI

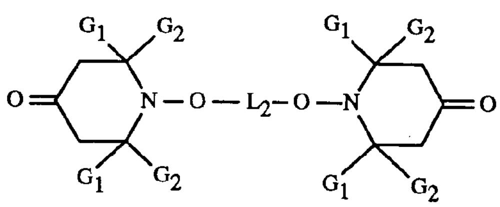

XVII

wherein

$G_1$and $G_2$ are independently alkyl of 1 to 4 carbon atoms, or $G_1$ and $G_2$ together are pentamethylene;

$L_1$ is alkyl of 1 to 18 carbon atoms, cycloalkyl of 5 to 12 carbon atoms, alkenyl of 2 to 18 carbon atoms, cycloalkenyl of 5 to 12 carbon atoms, aralkyl of 7 to 15 carbon atoms, a radical of a saturated or unsaturated bicyclic or tricyclic hydrocarbon of 7 to 12 carbon atoms or aryl of 6 to 10 carbon atoms or said aryl substituted by alkyl, or $L_1$ is $-CH_2CH_2COOL_3$ where $L_3$ is alkyl of 1 to 18 carbon atoms,

n is 1 or 2,

when n is 1,

R is hydrogen, $C_1$-$C_{18}$-alkyl optionally interrupted by one or more oxygen atoms, cyanoethyl, benzyl, glycidyl, a monovalent acyl radical of an aliphatic, cycloaliphatic, araliphatic or aromatic acid, or of carbamic acid or of a phosphorus-containing acid, or a monovalent silyl radical; or

when n is 2,

R is $C_1$-$C_{12}$-alkylene, $C_4$-$C_{12}$-alkenylene, xylylene, a divalent acyl radical of an aliphatic, cycloaliphatic, araliphatic or aromatic dicarboxylic acid, or of a dicarbamic acid or of a phosphorus-containing acid, or a bivalent silyl radical;

p is 1, 2 or 3,

$R_1$ is $C_1$-$C_{12}$-alkyl, $C_5$-$C_7$-cycloalkyl, $C_7$-$C_8$-arakyl, $C_2$-$C_{18}$-alkanoyl, $C_3$-$C_5$-alkenoyl or benzoyl;

when p is 1,

$R_2$ is $C_1$-$C_{18}$-alkyl, $C_5$-$C_7$-cycloalkyl, $C_2$-$C_8$-alkenyl unsubstituted or substituted by a cyano, carbonyl or carbamide group, or is glycidyl, a group of the formula $-CH_2CH(OH)$-Z or of the formula -CONH-Z wherein Z is hydrogen, methyl or phenyl; or

when p is 2,

$R_2$ is $C_2$-$C_{12}$-alkylene, $C_6$-$C_{12}$-arylene, xylylene, a $-CH_2CH(OH)CH_2$-O-X-O-$CH_2CH(OH)CH_2$- wherein X is $C_2$-$C_{10}$-alkylene, $C_6$-$C_{15}$-arylene or $C_6$-$C_{12}$-cycloalkylene; or, provided that $R_1$ is not alkanoyl, alkenoyl or benzoyl, $R_2$ can also be a divalent acyl radical of an aliphatic, cycloaliphatic or aromatic dicarboxylic acid or dicarbamic acid, or can be the group -CO-; or $R_1$ and $R_2$ together when p is I can be the cyclic acyl radical of an aliphatic or aromatic 1,2- or 1,3-dicarboxylic acid; or

$R_2$ is

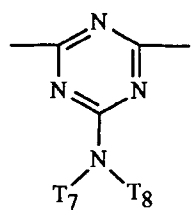

where $T_7$ and $T_8$ are independently hydrogen, alkyl of 1 to 18 carbon atoms, or $T_7$ and $T_8$ together are alkylene of 4 to 6 carbon atoms or 3-oxapentamethylene;

when p is 3,

$R_2$ is 2,4,6-triazinyl;

when n is 1,

$R_3$ is $C_2$-$C_8$-alkylene or hydroxyalkylene or $C_4$-$C_{22}$-acyloxyalkylene; or

when n is 2,

$R_3$ is $(-CH_2)_2C(CH_2-)_2$;

when n is 1,

$R_4$ is hydrogen, $C_1$-$C_{12}$-alkyl, $C_3$-$C_5$-alkenyl, $C_7$-$C_9$-aralkyl, $C_5$-$C_7$-cycloalkyl, $C_2$-$C_4$-hydroxyalkyl, $C_2$-$C_6$-alkoxyalkyl, $C_6$-$C_{10}$-aryl, glycidyl, a group of formula $-(CH_2)_m$-COO-Q or of the formula $-(CH_2)_m$-O-CO-Q wherein m is 1 or 2 and Q is $C_1$-$C_4$-alkyl or phenyl; or

when n is 2,

$R_4$ is $C_2$-$C_{12}$-alkylene, $C_6$-$C_{12}$-arylene, a group $-CH_2CH(OH)CH_2$-O-X-O-$CH_2CH(OH)CH_2$- wherein X is $C_2$-$C_{10}$-alkylene, $C_6$-$C_{15}$-arylene or $C_6$-$C_{12}$-cycloalkylene, or a group $-CH_2CH(OZ_1)CH_2-(OCH_2CH(OZ_1)CH_2)_2$- wherein $Z_1$ is hydrogen, $C_1$-$C_{18}$-alkyl, allyl, benzyl, $C_2$-$C_{12}$-alkanoyl or benzoyl;

$R_5$ is hydrogen, $C_1$-$C_{12}$-alkyl, allyl, benzyl, glycidyl or $C_2$-$C_6$-alkoxyalkyl;

$Q_1$ is $-N(R_7)$- or -O-;

E is $C_1$-$C_3$-alkylene, the group $-CH_2CH(R_8)$-O- wherein $R_8$ is hydrogen, methyl or phenyl, the group $-(CH_2)_3$-NH- or a direct bond;

$R_7$ is $C_1$-$C_{18}$-alkyl, $C_5$-$C_7$-cycloalkyl, $C_7$-$C_{12}$-aralkyl, cyanoethyl, $C_6$-$C_{10}$-aryl, the group $-CH_2CH(R_8)$-OH; or a group of the formula

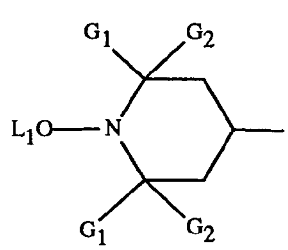

or a group of the formula

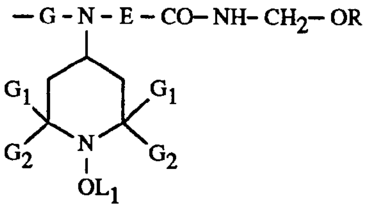

wherein G is $C_2$-$C_6$-alkylene or $C_6$-$C_{12}$-arylene; or
$R_7$ is a group -E-CO-NH-$CH_2$-$OR_6$;
$R_6$ is hydrogen or $C_1$-$C_{18}$-alkyl;
Formula VI denotes a recurring structural unit of a polymer where T is ethylene or 1,2-propylene, or is a repeating structural unit derived from an α-olefin copolymer with an alkyl acrylate or methacrylate;
k is 2 to 100;
$T_1$ has the same meaning as $R_2$ when p is 1 or 2;
M and Y are independently methylene or carbonyl;
$T_2$ has the same meaning as $R_4$,
$T_3$ and $T_4$ are independently alkylene of 2 to 12 carbon atoms, of $T_4$ is

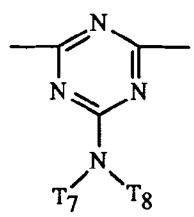

$T_6$ is

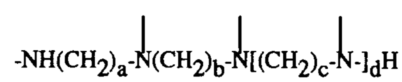

where a, b and c are independently 2 or 3, and d is 0 or 1;
e is 3 or 4;
$T_5$ is the same as R with the proviso that $T_5$ cannot be hydrogen when n is 1;
$E_1$ and $E_2$ being different, are each oxo or imino;
$E_3$ is hydrogen, alkyl of 1 to 30 carbon atoms, phenyl, naphthyl, said phenyl or said naphthyl substituted by chlorine or by alkyl of 1 to 4 carbon atoms, or phenylalkyl of 7 to 12 carbon atoms, or said phenylalkyl substituted by alkyl of 1 to 4 carbon atoms;
$E_4$ is hydrogen, alkyl of 1 to 30 carbon atoms, phenyl, naphthyl or phenylalkyl of 7 to 17 carbon atoms; or
$E_3$ and $E_4$ together are polymethylene of 4 to 17 carbon atoms, or said polymethylene substituted by up to four alkyl groups of 1 to 4 carbon atoms; and
$L_2$ is an alkanediyl of 1 to 18 carbon atoms or cyclohexanediyl.

**2.** A composition according to claim 1 wherein the effective amount of component (b) is 1 to 10000 ppm, preferably 5 to 2000 ppm and more preferably 50 to 1000 ppm, based on the total monomer composition.

**3.** A composition according to claim 1 wherein component (a) is a monomer selected from the group consisting of the olefinic hydrocarbons, dienes, halogenated monomers, unsaturated acids, unsaturated esters, unsaturated amides, unsaturated nitriles, unsaturated ethers, acrylated urethanes and unsaturated polyesters and mixtures thereof.

**4.** A composition according to claim 3 wherein the monomer is styrene, butadiene, vinyl chloride, acrylic acid, methacrylic acid, vinyl acetate, 2-hydroxyethyl acrylate, 2-hydroxyethyl methacrylate, trimethylolpropane

triacrylate, polyethylene glycol diacrylate or methyl methacrylate.

5. A composition according to claim 4 wherein the monomer is styrene, butadiene, acrylic acid or methacrylic acid.

6. A composition according to claim 1 wherein component (b) is of formula I or XV, preferably of formula I.

7. A composition according to claim 1 wherein component (b) is selected from the group consisting of
1-α-methylbenzyloxy-2,2,6,6-tetramethylpiperidin-4-one;
1-α-methylbenzyloxy-2,2,6,6-tetramethylpiperidin-4-ol;
1-α-methylbenzyloxy-2,2,6,6-tetramethyl-4,4-ethylenedioxypiperidine;
1-tert-butoxy-2,2,6,6-tetramethylpiperidin-4-ol;
1 -methylcyclohexyloxy-2,2,6,6-tetramethyl-4-benzoyloxypiperidine;
bis(1-octyloxy-2,2,6,6-tetramethylpiperidin-4-yl) sebacate;
1-[2-(methoxycarbonyl)ethoxy]-4-benzyloxy-2,2,6,6-tetramethylpiperidine; and
1-methoxy-4-benzoyloxy-2,2,6,6-tetramethylpiperidine.

8. A composition according to claim 1 which additionally contains another stabilizer selected from the group consisting of hydroquinone, monomethyl ether of hydroquinone and phenothiazine.

9. A process for preventing the premature polymerization of a monomer polymerizable by free radical initiation which comprises
adding to said monomer (a) an effective amount of component (b) according to claim 1.

10. A process according to claim 9 for preventing the fouling of processing equipment including reactors, pipes, stills, distillation columns, cracking towers and heat transfer surfaces during the processing of a monomer polymerizable by free radical intiation which comprises
adding to said monomer (a), before processing is begun, an effective amount of component (b) according to claim 1.

11. A process according to claim 9 which comprises
adding as component (b) 10 to 500 ppm of at least one compound of formula I to XVII to a continuous fluid feed stream to deactivate the autocatalytic polymerization, in any part of the continuous process equipment, of any ethylenically unsaturated monomer present in the feed stream, and
further adding to said feed stream an additional 10 ppb to 500 ppm of at least one compound of formula I to XVII as a makeup additive to maintain the desired concentration of said compound in the fluid feed stream being processed.

European Patent Office

# EUROPEAN SEARCH REPORT

Application Number
EP 91810562.8

## DOCUMENTS CONSIDERED TO BE RELEVANT

| Category | Citation of document with indication, where appropriate, of relevant passages | Relevant to claim | CLASSIFICATION OF THE APPLICATION (Int. Cl.5) |
|---|---|---|---|
| D,A | US - A - 4 668 721 (R. SELTZER et al.) * Column 3, line 45 - column 13, line 53; claims * -- | 1-3 | C 07 C 69/54<br>C 07 C 57/075<br>C 07 C 15/46<br>C 07 C 67/62<br>C 07 C 7/20 |
| D,A | US - A - 4 691 015 (R.A. BEHRENS et al.) * Column 1, line 45 - column 10, line 29; claims * -- | 1-3,8 | |
| D,A | DIE MAKROMOLEKULARE CHEMIE, vol. 160, October 18, 1972 Y. MIURA et al. "Vinyl Polymerization. 286. Inhibiting Effect of 2.2.6.6-Tetramethyl -4-oxo-piperidine-1-oxyl in Radical Polymerization" pages 243-249 * No. II, pages 244,245 * -- | 1-3 | TECHNICAL FIELDS SEARCHED (Int. Cl.5) |
| A | DE - B2 - 2 113 246 (SANKYO) * Claims; example * ---- | 1-5 | C 07 C 7/00<br>C 07 C 15/00<br>C 07 C 51/00<br>C 07 C 57/00<br>C 07 C 67/00<br>C 07 C 69/00 |

The present search report has been drawn up for all claims

| Place of search | Date of completion of the search | Examiner |
|---|---|---|
| VIENNA | 07-10-1991 | HOFBAUER |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons

& : member of the same patent family, corresponding document

EPO FORM 1503 03.82 (P0401)